# EUROPÄISCHE PATENTSCHRIFT

(11) **EP 2 721 933 B1**
(45) Veröffentlichungstag und Bekanntmachung des Hinweises auf die Patenterteilung: **09.08.2017**
(21) Anmeldenummer: 13188893.5
(22) Anmeldetag: 16.10.2013
(51) Int. Cl.: A23L 2/52, A23L 2/385, A23L 29/25, A61K 8/34, A61K 8/60, A61K 8/67, A61K 8/73, A61Q 19/00, A61K 9/00, A23L 33/15, A23L 5/44

(54) **Emulsion**
Emulsion
Emulsion

(30) Priorität: 16.10.2012 EP 12188663
(43) Veröffentlichungstag der Anmeldung: 23.04.2014
(73) Patentinhaber: Döhler GmbH, 64295 Darmstadt (DE)
(72) Erfinder: Wydra, Markus, Dr., 64673 Zwingenberg (DE); Lukanowski, Johann, 64319 Pfungstadt (DE); Leicht, Alexander, 64404 Bickenbach (DE); Thomas, Christine, 64295 Darmstadt (DE)
(74) Vertreter: dompatent von Kreisler Selting Werner - Partnerschaft von Patent- und Rechtsanwälten mbB

(56) Entgegenhaltungen:
- EP-A1- 2 359 702
- WO-A1-2013/135759
- US-A- 4 568 667
- US-A- 6 013 255

## Beschreibung

Die vorliegende Erfindung betrifft eine Emulsion, insbesondere zur Herstellung von Getränken und ein Verfahren zur Herstellung der Emulsion und der Getränke.

Es ist wünschenswert, in flüssige, wässrige Lebensmittel, insbesondere Getränke, aber auch in Futtermittel, pharmazeutische und kosmetische Produkte hydrophobe Substanzen, beispielsweise Farbstoffe, Vitamine oder physiologisch wirksame Inhaltsstoffe, einzubringen.

Hierzu werden entsprechende Substanzen zumeist in Lipiden, beispielsweise pflanzlichen Ölen, gelöst und in dem Getränk dispergiert. Hierbei ist es wichtig, dass die Substanzen zum einen auch bei längerfristiger Lagerung dispergiert bleiben und sich die Lipidphase nicht absetzt. Weiterhin ist es wünschenswert, dass das Getränk mit der entstandenen Emulsion klar ist, da entsprechende Produkte vom Verbraucher besser akzeptiert werden.

Da Getränke zumeist aus Grundstoffzubereitung hergestellt werden, ist es weiterhin wünschenswert, dass die Emulsionen leicht zu dosieren sind und eine hohe mechanische und thermische Stabilität und Säurestabilität aufweisen. Auch eine Stabilität gegen Enzyme ist wünschenswert. Sie sollen darüber hinaus bevorzugt einen neutralen Geschmack aufweisen.

Emulgatorsysteme für diesen Zweck sind dem Fachmann bekannt. Trotzdem besteht weiterhin Bedarf nach neuen Emulsionen, die die zahlreichen oben genannten Anforderungen möglichst optimal erfüllen und zumindest in einigen Bereichen dem Stand der Technik überlegen sind.

Aufgabe der vorliegenden Erfindung war es, Emulsionen bereitzustellen, die zumindest einige der Nachteile des Standes der Technik überwinden können.

Gelöst wird die Aufgabe durch eine Emulsion enthaltend neben Wasser
- 1 bis 50 Gew.-%, bezogen auf die Emulsion, einer Lipidphase
- 1 bis 10 Gew.-%, bezogen auf die Emulsion, Saponine
- 5 bis 40 Gew.-%, bezogen auf die Emulsion, Polyole
- 5 bis 60 Gew.-%, bezogen auf die Emulsion, Kohlenhydrate
- 1 bis 30 Gew.-%, bezogen auf die Emulsion, Hydrokolloide, wobei die Hydrokolloide aus der Gruppe bestehend aus Gummi Arabicum, modifiziertes Gummi arabicum, Gum ghatti, modifiziertes Gum ghatti und Mischungen davon ausgewählt werden
- 0 bis 10 Gew.-%, bezogen auf die Emulsion, sonstige Inhaltsstoffe.

Alternativ gelöst wird die Aufgabe durch eine Emulsion enthaltend neben Wasser
- 1 bis 50 Gew.-%, bezogen auf die Emulsion, einer Lipidphase
- 1 bis 10 Gew.-%, bezogen auf die Emulsion, Saponine
- 5 bis 40 Gew.-%, bezogen auf die Emulsion, Polyole
- 5 bis 40 Gew.-%, bezogen auf die Emulsion, Kohlenhydrate
- 1 bis 30 Gew.-%, bezogen auf die Emulsion, Hydrokolloide, wobei die Hydrokolloide aus der Gruppe bestehend aus Gummi Arabicum, modifiziertes Gummi arabicum, Gum ghatti, modifiziertes Gum ghatti und Mischungen davon ausgewählt werden
- 0 bis 10 Gew.-%, bezogen auf die Emulsion, sonstige Inhaltsstoffe.

Als Saponine eignen sich insbesondere pflanzliche Sterolglykoside. Diese können beispielsweise aus Yucca schidgera, Yucca valida und besonders bevorzugt aus Quillaja saponaria gewonnen werden. Molekulargewichte im Bereich von 1500 bis 2500 Da sind besonders geeignet. Bevorzugt sind die Saponine nicht-ionisch. Mengen von 1 bis 5 Gew.-% bezogen auf die Emulsion sind besonders bevorzugt.

Als Polyole eignen sich insbesondere Polyole ausgewählt aus der Gruppe bestehend aus 1,2-Propandiol, Glycerin, Monoester des Glycerins mit C1-C5-Monocarbonsäuren, Monoether des Glycerins, Sorbitol und Mischungen davon. Mengen von 15 bis 25 Gew.-% bezogen auf die Emulsion sind besonders bevorzugt.

Als Kohlenhydrate werden bevorzugt niedermolekulare Zucker eingesetzt mit bevorzugt 1 oder 2 Monosaccharideinheiten. Besonders geeignete Zucker sind Invertzucker, Saccharose, Fructose, Glucose, High Fructose Corn Syrup, Isomaltulose, Trehalose und Mischungen davon. Mengen von 10 bis 60 Gew.-%, von 5 bis 40 Gew.-% oder von 15 bis 25 Gew.-% jeweils bezogen auf die Emulsion sind besonders bevorzugt.

Als Hydrokolloide eignen sich die Substanzen aus der Gruppe bestehend aus Gummi arabicum, modifiziertes Gummi arabicum, Gum ghatti, modifiziertes Gum ghatti und Mischungen davon. Mengen von 2 bis 10 Gew.-% bezogen auf die Emulsion sind besonders bevorzugt.

Als Lipide für die Lipidphase eignen sich insbesondere Lipide aus der Gruppe bestehend aus Sonnenblumenöl, Kokosöl, Triglyceride von mittelkettigen Fettsäuren, Maiskeimöl, Reiskeimöl, Rapsöl, Erdnussöl, Palmöl, als auch Terpene wie Citrus- und Orangenterpene, und Mischungen davon. Mengen von 3 bis 10 Gew.-% bezogen auf die Emulsion sind besonders bevorzugt.

Der Wasseranteil, bezogen auf die Emulsion, liegt bevorzugt im Bereich von 10 bis 70 Gew.-%, besonders bevorzugt von 40 bis 60 Gew.-%.

In einer bevorzugten Ausführungsform der Erfindung enthält die Emulsion bereits einen gelösten hydrophoben Wirkstoff. Dieser liegt bevorzugt in einer Konzentration im Bereich von 0,1 bis 70 Gew.-% bezogen auf die Lipidphase vor, bevorzugt 5 bis 20 Gew.-%. Wenn also erfindungsgemäß 1 bis 50 Gew.-% einer Lipidphase vorhanden sind, kann diese Lipidphase beispielsweise 10 bis 50 Gew.-% eines hydrophoben Wirkstoffs und entsprechend 90 bis 50 Gew.-% Lipide, beispielsweise die oben genannten Lipide, enthalten.

Die erfindungsgemäß definierte Lipidphase umfasst somit sowohl Lipide und/oder Terpene als auch die in ihr gelösten oder mikroskopisch klein dispergierten Wirkstoffe.

Bevorzugte hydrophobe Wirkstoffe sind beispielsweise Farbstoffe, Vitamine, funktionelle, lipophile Ingredenzien, sekundäre Pflanzenstoffe. Beispiele hierfür sind Astaxanthin, Canthaxanthin, Zeaxanthin, beta-Carotin, Mischungen aus α- und ß-Carotin, apo-Carotinal, Lycopin, Lutein und Luteinester, Kupfer-Chlorophyll, Kupfer-Chlorophyllin, Curcumin oder Paprikaextrakt, mehrfach ungesättigte Fettsäuren, alpha-Tocopherol, gemischte Tocopherole, Tocotrienole, Xanthohumol, Resveratrol, Vitamin D, Vitamin K und Mischungen davon.

Bezogen auf die gesamte Emulsion kann der Gehalt an hydrophoben Wirkstoffen, die in der Lipidphase gelöst sind, bevorzugt im Bereich von 0,001 bis 35 Gew.-% liegen, besonders bevorzugt im Bereich von 10 bis 20 Gew.-%.

Weiterhin kann die Emulsion weitere Inhaltsstoffe enthalten. Hierzu zählen insbesondere Säuerungs-, Antioxidations- und Konservierungsstoffe, aber auch weitere wasserlösliche Farbstoffe. Bevorzugte und in diesem Bereich übliche Zusatzstoffe sind Genußsäuren, wie Citronensäure, und Stabilisatoren wie Tocopherole, Ascorbyl-Palmitat, Ascorbinsäure, Kaliumsorbat und Natriumbenzoat.

Erfindungsgemäß handelt es sich um Emulsionen, deren Verwendung in zu konsumierenden Produkten wie z.B. einem Fertiggetränk zu einem klaren, transparenten Produkt führt. Solche Produkte mit Dosagen der jeweiligen Emulsion von 0,01 bis 5 g/L, alternativ von 0,01 bis 5%, weisen also eine Trübung unterhalb von 20 FNU auf. FNU wird nephelometrisch gemessen.

Gegenstand der Erfindung ist auch ein Verfahren zur Herstellung einer Emulsion umfassend das Vermischen von Wasser mit
- 1 bis 50 Gew.-%, bezogen auf die Emulsion, einer Lipidphase
- 1 bis 10 Gew.-%, bezogen auf die Emulsion, Saponine
- 5 bis 40 Gew.-%, bezogen auf die Emulsion, Polyole
- 5 bis 60 Gew.-%, bezogen auf die Emulsion, Kohlenhydrate
- 1 bis 30 Gew.-%, bezogen auf die Emulsion, Hydrokolloide, wobei die Hydrokolloide aus der Gruppe bestehend aus Gummi Arabicum, modifiziertes Gummi arabicum, Gum ghatti, modifiziertes Gum ghatti und Mischungen davon ausgewählt werden
- 0 bis 10 Gew.-%, bezogen auf die Emulsion, sonstige Inhaltsstoffe.

Alternativer Gegenstand der Erfindung ist auch ein Verfahren zur Herstellung einer Emulsion umfassend das Vermischen von Wasser mit
- 1 bis 50 Gew.-%, bezogen auf die Emulsion, einer Lipidphase
- 1 bis 10 Gew.-%, bezogen auf die Emulsion, Saponine
- 5 bis 40 Gew.-%, bezogen auf die Emulsion, Polyole
- 5 bis 40 Gew.-%, bezogen auf die Emulsion, Kohlenhydrate
- 1 bis 30 Gew.-%, bezogen auf die Emulsion, Hydrokolloide, wobei die Hydrokolloide aus der Gruppe bestehend aus Gummi Arabicum, modifiziertes Gummi arabicum, Gum ghatti, modifiziertes Gum ghatti und Mischungen davon ausgewählt werden
- 0 bis 10 Gew.-%, bezogen auf die Emulsion, sonstige Inhaltsstoffe.

Für die Kohlenhydrate sind Mengen von 10 bis 60 Gew.-%, von 5 bis 40 Gew.-% oder von 15 bis 25 Gew.-% jeweils bezogen auf die Emulsion besonders bevorzugt.

Bevorzugt erfolgt ein Voremulgieren, beispielsweise in einem Rotorstatorsystem mit anschließender Homogenisierung in einem Hochdruckhomogenisator. Drücke im Bereich von 300 bis 1000 bar können hierfür erfolgreich eingesetzt werden.

Die erfindungsgemäße Emulsion kann beispielsweise verwendet werden um ein Getränk zu erhalten. Daher ist Gegenstand der Erfindung auch ein Getränk erhältlich durch Vermischen von 0,01 bis 5 g/L der erfindungsgemäßen Emulsion mit Wasser und/oder weiteren Getränkegrundstoffen.

Daher ist ein alternativer Gegenstand der Erfindung auch ein Getränk erhältlich durch Vermischen von 0,01 bis 5% der erfindungsgemäßen Emulsion mit Wasser und/oder weiteren Getränkegrundstoffen.

Gegenstand der Erfindung ist auch ein Verfahren zur Herstellung eines Getränkes umfassend das Vermischen von 0,01 bis 5 g/L einer erfindungsgemäßen Emulsion und weiteren Inhaltsstoffen und/oder Wasser.

Alternativer Gegenstand der Erfindung ist auch ein Verfahren zur Herstellung eines Getränkes umfassend das Vermischen von 0,01 bis 5 Gew.-% einer erfindungsgemäßen Emulsion und weiteren Inhaltsstoffen und/oder Wasser Bevorzugt ist dieses Getränk klar, hat also eine Trübung von unter 20 FNU, bevorzugt unter 10 FNU.

Die erfindungsgemäßen Getränke sind bei mechanischer, thermischer Belastung und Langzeitlagerung stabil. Selbst oxidativer Stress (Sauerstoff- oder Lichtbelastung) führen weder zu einer Zerstörung der Emulsion noch zu einer Zerstörung eines daraus hergestellten Getränkes.

Während konventionelle Emulsionen zumeist trübe Getränke hervorbringen, ist mit der erfindungsgemäßen Emulsion die Herstellung von klaren Getränken möglich.

Die erfindungsgemäßen Emulsionen können auch in Grundstoffe eingebracht werden, die dann zu Getränken ausgemischt werden.

Gegenstand der Erfindung ist auch ein Getränkegrundstoff, ein Futtermittel, ein pharmazeutisches Produkt und ein kosmetisches Produkt, dass die erfindungsgemäße Emulsion enthält.

Trotz des hohen Ölgehalts (bis zu 50% der Emulsion, die in einer Menge von bis zu 0,5%, alternativ 5%, in das Getränk eingebracht wird), ist dieses ohne den Einsatz von Beschwerungsmitteln stabil, d.h. es erfolgt keine Bildung eines Ölrings im Getränk.

Bevorzugt wird auf den Einsatz von Beschwerungsmitteln verzichtet.

Im Gegensatz zu vielen pulverförmigen Formulierungen für hydrophobe Wirkstoffe sind die erfindungsgemäßen Emulsionen aufgrund der flüssigen Form leichter dosierbar.

Gegenüber anderen z.B. Zuckerester-basierten System, beispielsweise Saccharosemonoestern von Speisefettsäuren, sind die erfindungsgemäßen Systeme auch bei einer Lagerung bei 60°C für 48 h stabil. Viele Zuckerester-basierte Systeme des Standes der Technik zeigen eine mäßige bzw. deutlich limitierte pH-Stabilität, während die erfindungsgemäßen Emulsionen im Getränk üblichen pH-Bereich von 2 bis 7 nicht zur Trübung oder Bildung von Flocken führen.

Im Gegensatz zum Beispiel von Polysorbat-stabilisierten Systemen haben sie einen neutralen Geschmack.

Überraschenderweise zeigen die erfindungsgemäßen Zusammensetzungen eine bessere Stabilität gegenüber enzymatischen Angriffen. Enzymatische Angriffe umfassen grundsätzlich sowohl amylolytische als auch proteolytische Angriffe. Die Amylasen oder Proteasen können auf verschiedenen Wegen in die Emulsion oder Getränke gelangen.

Zum einen können eingesetzte Rohstoffe aufgrund ihres natürlichen Ursprungs Enzyme enthalten. Dies gilt beispielweise für Gerstenmalzextrakt, der in alkoholfreien Bieren eingesetzt wird.

Auch bei der Herstellung von Gemüsesäften, Fruchtsäften oder Zusatzstoffen können Enzyme als Prozesshilfsmittel eingesetzt werden. Solche Einsätze müssen nicht deklariert werden. Der Endabfüller ist daher über eventuelle Restaktivitäten nicht informiert.

Grundsätzlich ist natürlich auch eine Kontamination durch Enzyme beispielsweise nach mikrobiellem Befall (vor allem durch Schimmel) zu befürchten.

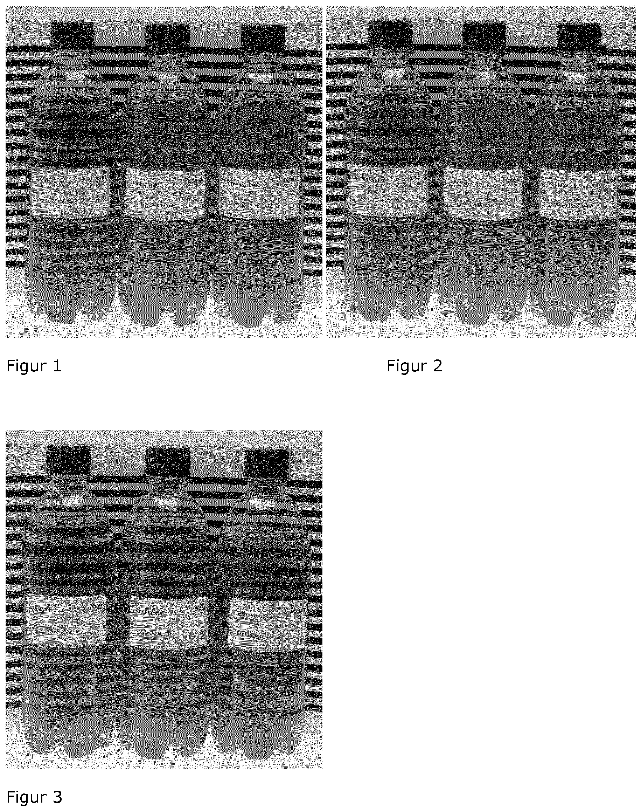

Die Figuren 1 bis 3 zeigen Untersuchungen zur enzymatischen Stabilität gemäß Beispiel 4.

Die erfindungsgemäßen Zusammensetzungen werden durch die folgenden Beispiele näher erläutert:

### Beispiel 1: beta-Carotin Emulsion

| **Position** | **Inhaltsstoff** | **Gew.-Anteil (%)** |
|---|---|---|
| 1 | Kaliumsorbat, kristallin | 0,10 |
| 2 | Zitronensäure, kristallin | 0,15 |
| 3 | Ascorbinsäure, kristallin | 0,20 |
| 4 | Quillajasaponin, als Trockenmasse | 2,50 |
| 5 | Gummi arabicum Pulver | 4,00 |
| 6 | Propylenglykol | 20,00 |
| 7 | Invertzuckersirup, 65 °Bx | 20,00 |
| 8 | Wasser | 44,05 |
| 9 | Beta-Carotin, 30%ig in Sonnenblumenöl | 4,30 |
| 10 | Orangenterpene | 4,50 |
| 11 | Tocopherol | 0,20 |

### Beispiel 2: Vitamin-E-Emulsion

| **Position** | **Inhaltsstoff** | **Gew.-Anteil (%)** |
|---|---|---|
| 1 | Kaliumsorbat, kristallin | 0,10 |
| 2 | Zitronensäure, kristallin | 0,15 |
| 3 | Ascorbinsäure, kristallin | 0,20 |
| 4 | Quillajaextrakt, flüssig | 2,50 |
| 5 | Gummi arabicum Pulver | 4,00 |
| 6 | Propylenglykol | 20,00 |
| 7 | Invertzuckersirup, 65 °Bx | 20,00 |
| 8 | Wasser | 45,40 |
| 9 | Vitamin E | 5,65 |
| 10 | Orangenterpene | 2,00 |

Aus der Emulsion gemäß Beispiel 1 und 2 konnte durch folgende Zusätze ein klares Getränk mit hohem Anteil an beta-Carotin bzw. Vitamin E hergestellt werden.

### Beispiel 3: Getränk

| **Position** | **Inhaltsstoff** | **Gew.-Anteil (%)** |
|---|---|---|
| 1 | Invertzuckersirup, 65 °Bx | 15,4 |
| 2 | Zitronensäure, kristallin | 0,2 |
| 3 | Ascorbinsäure, kristallin | 0,02 |
| 4 | Emulsion Beispiel 1 oder 2 | 0,05 |
| 5 | Wasser | 84,33 |

### Beispiel 4: Enzymatische Stabilität

Zur Untersuchung der enzymatischen Stabilität wurden drei Rezepturen hergestellt; siehe die nachfolgenden Tabellen.

Die Ölphase wurde dazu auf 130°C erhitzt und unter Scherung eines Rotor-Stator Systems zu einer Voremulsion verarbeitet. Diese Emulsion wurde mittels eines Hochdruckhomogenisators bei 800/80 bar mehrfach homogenisiert.

**Zusammensetzung Emulsion A (Vergleichsbeispiel)**

| **Position** | **Inhaltsstoff** | **Anteil (%)** |
|---|---|---|
| 1 | Kaliumsorbat, kristallin | 0,10 |
| 2 | Zitronensäure, kristallin | 0,15 |
| 3 | Ascorbinsäure, kristallin | 0,20 |
| *4* | *Quillajaextrakt, Trockenmasse* | *2,50* |
| 5 | Propylenglykol | 20,00 |
| 6 | Invertzuckersirup, 65 °Bx | 20,00 |
| 7 | Wasser | 48,05 |
| 8 | Beta-Carotin, 30%ig in Sonnenblumenöl | 4,30 |
| 9 | Orangenterpene | 4,50 |
| 10 | Tocopherol | 0,20 |

**Zusammensetzung Emulsion B (Vergleichsbeispiel)**

| **Position** | **Inhaltsstoff** | **Anteil (%)** |
|---|---|---|
| 1 | Kaliumsorbat, kristallin | 0,10 |
| 2 | Zitronensäure, kristallin | 0,15 |
| 3 | Ascorbinsäure, kristallin | 0,20 |
| *4* | *Quillajaextrakt, Trockenmasse* | *2,50* |
| *5* | *modifizierte Stärke Stärkenatriumoctenylsuccinat* | *5,62* |
| 6 | Propylenglykol | 20,00 |
| 7 | Invertzuckersirup, 65 °Bx | 20,00 |
| 8 | Wasser | 42,43 |
| 9 | Beta-Carotin, 30%ig in Sonnenblumenöl | 4,30 |
| 10 | Orangenterpene | 4,50 |
| 11 | Tocopherol | 0,20 |

**Zusammensetzung Emulsion C**

| **Position** | **Inhaltsstoff** | **Anteil (%)** |
|---|---|---|
| 1 | Kaliumsorbat, kristallin | 0,10 |
| 2 | Zitronensäure, kristallin | 0,15 |
| 3 | Ascorbinsäure, kristallin | 0,20 |
| *4* | *Quillajaextrakt, Trockenmasse* | *2,50* |
| *5* | *Gummi arabicum Pulver* | *7,50* |
| 6 | Propylenglykol | 20,00 |
| 7 | Invertzuckersirup, 65 °Bx | 20,00 |
| 8 | Wasser | 40,55 |
| 9 | Beta-Carotin, 30%ig in Sonnenblumenöl | 4,30 |
| 10 | Orangenterpene | 4,50 |
| 11 | Tocopherol | 0,20 |

Aus den Emulsionen A bis C wurden Fertiggetränke hergestellt und in 0,5 L PET Flaschen abgefüllt. Die Zusammensetzung des Getränks ist in der nachfolgenden Tabelle enthalten:

**Zusammensetzung der Getränke**

| **Position** | **Inhaltsstoff** | **Anteil (%)** |
|---|---|---|
| 1 | Invertzuckersirup, 65 °Bx | 15,40 |
| 2 | Zitronensäure, kristallin | 0,20 |
| 3 | Ascorbinsäure, kristallin | 0,02 |
| 4 | Emulsion | 0,05 |
| 5 | Wasser | 84,33 |

Je zwei Flaschen wurde mit je 150 µL einer Enzymlösung versetzt (amylolytisches Enzym: Beerzym ALPHA-BETA, Erbslöh, Geisenheim bzw. proteolytisches Enzym: Corolase 7089, AB Enzymes, Darmstadt) und bei Raumtemperatur und Tageslicht inkubiert. Die Proben wurden über 4 Tage beobachtet. Als Kontrolle diente eine Flasche ohne Zusatz.
Figur 1 zeigt die Inkubation des Getränks aus der Emulsion A ohne Enzym (links), mit Amylasezusatz (Mitte) und mit Proteasezusatz (rechts).
Figur 2 zeigt die Inkubation des Getränks aus der Emulsion B ohne Enzym (links), mit Amylasezusatz (Mitte) und mit Proteasezusatz (rechts).
Figur 3 zeigt die Inkubation des Getränks aus der Emulsion C ohne Enzym (links), mit Amylasezusatz (Mitte) und mit Proteasezusatz (rechts).

Eine Störung der Emulsion im Getränk (Entmischung) zeigt sich zunächst an einer Trübung und anschließend an einer Ringbildung von Öl.

Bei dem Getränk mit der Emulsion A zeigt sich unter Einwirkung des amylolytischen Enzyms eine deutliche Trübung. In Gegenwart des proteolytischen Enzyms bricht die Emulsion zusammen.

Bei dem Getränk mit der Emulsion B zeigt sich in beiden Testsystemen bereits nach zweistündiger Inkubation eine Trübung und eine Ringbildung nach 24 Stunden.

Bei der Emulsion C blieben die Zubereitungen auch bei 96 stündiger Inkubation stabil.

## Patentansprüche

1. Emulsion enthaltend neben Wasser
• 1 bis 50 Gew.-%, bezogen auf die Emulsion, einer Lipidphase
• 1 bis 10 Gew.-%, bezogen auf die Emulsion, Saponine
• 5 bis 40 Gew.-%, bezogen auf die Emulsion, Polyole
• 5 bis 60 Gew.-%, bezogen auf die Emulsion, Kohlenhydrate
• 1 bis 30 Gew.-%, bezogen auf die Emulsion, Hydrokolloide, wobei die Hydrokolloide aus der Gruppe bestehend aus Gummi Arabicum, modifiziertes Gummi arabicum, Gum ghatti, modifiziertes Gum ghatti und Mischungen davon ausgewählt sind
• 0 bis 10 Gew.-%, bezogen auf die Emulsion, sonstige Inhaltsstoffe.

2. Emulsion nach Anspruch 1, wobei das Saponin ein pflanzliches Sterolglykosid ist.

3. Emulsion nach Anspruch 1 oder 2, wobei die Polyole aus der Gruppe bestehend aus 1,2-Propandiol, Glycerin, Monoester des Glycerins mit C1-C5-Monocarbonsäuren, Monoether des Glycerins, Sorbitol und Mischungen davon ausgewählt sind.

4. Emulsion nach einem der Ansprüche 1 bis 3, wobei die Kohlenhydrate aus der Gruppe bestehend aus Invertzucker, Saccharose, Fructose, Glucose, High Fructose Corn Syrup, Isomaltulose, Trehalose und Mischungen davon ausgewählt sind.

5. Emulsion nach einem der Ansprüche 1 bis 4, wobei die Menge an Kohlenhydraten 5 bis 40 Gew.-%, 15 bis 25 Gew.-% oder 10 bis 60 Gew.-%, bezogen auf die Emulsion, beträgt.

6. Emulsion nach einem der Ansprüche 1 bis 5, wobei die Lipide der Lipidphase aus der Gruppe bestehend aus Sonnenblumenöl, Kokosöl, Triglycerid von mittelkettigen Fettsäuren, Maiskeimöl, Reiskeimöl, Rapsöl, Erdnussöl, Palmöl, Citrusterpene, Orangenterpene und Mischungen davon ausgewählt sind.

7. Emulsion nach einem Ansprüche 1 bis 6, wobei in der Lipidphase ein hydrophober Wirkstoff in einer Konzentration 0,1 bis 70 Gew.-%, bezogen auf die Lipidphase, gelöst oder dispergiert ist.

8. Emulsion nach einem der Ansprüche 1 bis 7, wobei der hydrophobe Wirkstoff ein Farbstoff oder ein Wirkstoff ist, insbesondere aus der Gruppe bestehend aus Vitaminen, sekundären Pflanzenstoffen, Astaxanthin, Canthaxanthin, Zeaxanthin, beta-Carotin, Mischungen aus α- und β-Carotin, apo-Carotinal, Lycopin, Lutein, Kupfer-Chlorophyll, Kupfer-Chlorophyllin, Curcumin oder Paprikaextrakt, mehrfach ungesättigte Fettsäuren, alpha-Tocopherol, Tocotrienole, Xanthohumol, Resveratrol, Vitamin D, Vitamin K und Mischungen ausgewählt ist.

9. Emulsion nach einem der Ansprüche 1 bis 8, wobei die sonstigen Inhaltsstoffe ausgewählt sind aus Säuerungsmitteln, Antioxidationsmitteln und Konservierungsstoffen, besonders Citronensäure, Tocopherole, Ascorbyl-Palmitat, Ascorbinsäure, Kaliumsorbat und Natriumbenzoat.

10. Verfahren zur Herstellung einer Emulsion umfassend das Vermischen und Dispergieren von Wasser
• 1 bis 50 Gew.-%, bezogen auf die Emulsion, einer Lipidphase
• 1 bis 10 Gew.-%, bezogen auf die Emulsion, Saponine
• 5 bis 40 Gew.-%, bezogen auf die Emulsion, Polyole
• 5 bis 60 Gew.-%, bezogen auf die Emulsion, Kohlenhydrate, bevorzugt 5 bis 40 oder 10 bis 60 Gew.-%
• 1 bis 30 Gew.-%, bezogen auf die Emulsion, Hydrokolloide, wobei die Hydrokolloide aus der Gruppe bestehend aus Gummi Arabicum, modifiziertes Gummi arabicum, Gum ghatti, modifiziertes Gum ghatti und Mischungen davon ausgewählt sind
• 0 bis 10 Gew.-%, bezogen auf die Emulsion, sonstige Inhaltsstoffe.

11. Getränk erhältlich durch Vermischen von 0,01 bis 5 g/L der Emulsion gemäß einem der Ansprüche 1 bis 9 mit Wasser sowie gegebenenfalls weiteren Inhaltsstoffen.

12. Getränk nach Anspruch 11, **dadurch gekennzeichnet, dass** das Getränk klar ist, also eine Trübung unter 20 FNU aufweist.

13. Verfahren zur Herstellung eines Getränkes nach einem der Ansprüche 11 bis 12 umfassend das Vermischen von 0,01 bis 5 g/L einer Emulsion nach einem der Ansprüche 1 bis 9 und weiteren Inhaltsstoffen.

14. Getränkegrundstoff, Futtermittel, pharmazeutisches Produkt oder kosmetisches Produkt, enthaltend die Emulsion nach einem der Ansprüche 1 bis 9.

## Claims

1. An emulsion comprising, in addition to water:
• from 1 to 50% by weight, based on the emulsion, of a lipid phase;
• from 1 to 10% by weight, based on the emulsion, of saponins;
• from 5 to 40% by weight, based on the emulsion, of polyols;
• from 5 to 60% by weight, based on the emulsion, of carbohydrates;
• from 1 to 30% by weight, based on the emulsion, of hydrocolloids, wherein said hydrocolloids are selected from the group consisting of gum arabic, modified gum arabic, ghatti gum, modified ghatti gum, and mixtures thereof;
• from 0 to 10% by weight, based on the emulsion, of other ingredients.

2. The emulsion according to claim 1, wherein said saponin is a vegetable sterol glycoside.

3. The emulsion according to claim 1 or 2, wherein said polyols are selected from the group consisting of 1,2-propanediol, glycerol, monoesters of glycerol with C₁-C₅ monocarboxylic acids, monoethers of glycerol, sorbitol, and mixtures thereof.

4. The emulsion according to any of claims 1 to 3, wherein said carbohydrates are selected from the group consisting of invert sugar, sucrose, fructose, glucose, high fructose corn syrup, isomaltulose, trehalose, and mixtures thereof.

5. The emulsion according to any of claims 1 to 4, wherein the amount of carbohydrates is from 5 to 40% by weight, from 15 to 25% by weight, or from 10 to 60% by weight, based on the emulsion.

6. The emulsion according to any of claims 1 to 5, wherein the lipids of the lipid phase are selected from the group consisting of sunflower oil, coconut oil, triglyceride of medium-chain fatty acids, maize-germ oil, rice-germ oil, rapeseed oil, peanut oil, palm oil, citrus terpenes, orange terpenes, and mixtures thereof.

7. The emulsion according to any of claims 1 to 6, wherein a hydrophobic active ingredient is dissolved or dispersed in the lipid phase at a concentration of from 0.1 to 70% by weight, based on the lipid phase.

8. The emulsion according to any of claims 1 to 7, wherein said hydrophobic active ingredient is a colorant or an active ingredient selected, in particular, from the group consisting of vitamins, secondary phytochemicals, astaxanthin, canthaxanthin, zeaxanthin, beta-carotene, mixtures of α- and β-carotenes, apo-carotinal, lycopene, lutein, copper chlorophyll complex, copper chlorophyllin complex, curcumin or bell pepper extract, polyunsaturated fatty acids, alpha-tocopherol, tocotrienols, xanthohumol, resveratrol, vitamin D, vitamin K, and mixtures thereof.

9. The emulsion according to any of claims 1 to 8, wherein said other ingredients are selected from acidifiers, antioxidants and preservatives, especially citric acid, tocopherols, ascorbyl palmitate, ascorbic acid, potassium sorbate, and sodium benzoate.

10. A process for producing an emulsion, comprising the mixing and dispersing of water,
• from 1 to 50% by weight, based on the emulsion, of a lipid phase;
• from 1 to 10% by weight, based on the emulsion, of saponins;
• from 5 to 40% by weight, based on the emulsion, of polyols;
• from 5 to 60% by weight, based on the emulsion, of carbohydrates, preferably from 5 to 40 or from 10 to 60% by weight;
• from 1 to 30% by weight, based on the emulsion, of hydrocolloids, wherein said hydrocolloids are selected from the group consisting of gum arabic, modified gum arabic, ghatti gum, modified ghatti gum, and mixtures thereof;
• from 0 to 10% by weight, based on the emulsion, of other ingredients.

11. A beverage obtainable by mixing from 0.01 to 5 g/l of the emulsion according to any of claims 1 to 9 with water and optionally further ingredients.

12. The beverage according to claim 11, **characterized in that** said beverage is clear, i.e., has a turbidity below 20 FNU.

13. A process for producing a beverage according to either of claims 11 to 12, comprising the mixing of from 0.01 to 5 g/l of an emulsion according to any of claims 1 to 9 and further ingredients.

14. A beverage base, feed, pharmaceutical product or cosmetic product comprising the emulsion according to any of claims 1 to 9.

## Revendications

1. Émulsion contenant, en plus de l'eau :
• 1 à 50 % en poids, mesurés par rapport à l'émulsion, d'une phase lipidique ;
• 1 à 10 % en poids, mesurés par rapport à l'émulsion, de saponine ;
• 5 à 40 % en poids, mesurés par rapport à l'émulsion, de polyols ;
• 5 à 60 % en poids, mesurés par rapport à l'émulsion, d'hydrates de carbone ;
• 1 à 30 % en poids, mesurés par rapport à l'émulsion, d'hydrocolloïdes, selon laquelle les hydrocolloïdes sont sélectionnés à partir du groupe constitué par la gomme arabique, la gomme arabique modifiée, la gomme ghatti, la gomme ghatti modifiée et des mélanges de ces composants ;
• 0 à 10 % en poids, mesurés par rapport à l'émulsion, de divers ingrédients.

2. Émulsion selon la revendication 1, selon laquelle la saponine est un glycoside de stérol d'origine végétale.

3. Émulsion selon la revendication 1 ou 2, selon laquelle les polyols sont sélectionnés à partir du groupe constitué par le 1,2-propane diol, la glycérine, un monoester de glycérine avec des acides monocarboxyliques de type C1 à C5, un monoéther de glycérine, le sorbitol et des mélanges de ces composants.

4. Émulsion selon l'une des revendications 1 à 3, selon laquelle les hydrates de carbone sont sélectionnés à partir du groupe constitué par le sucre inverti, le saccharose, le fructose, le glucose, le sirop de maïs à haute teneur en fructose, l'isomaltulose, le tréhalose et des mélanges de ces composants.

5. Émulsion selon l'une des revendications 1 à 4, selon laquelle la quantité d'hydrates de carbone correspond à 5 à 40 % en poids ou 15 à 25 % en poids ou 10 à 60 % en poids, mesurés par rapport à l'émulsion.

6. Émulsion selon l'une des revendications 1 à 5, selon laquelle les lipides de la phase lipidique sont sélectionnés à partir du groupe constitué par l'huile de tournesol, l'huile de noix de coco, un triglycéride d'acides gras à chaîne moyenne, l'huile de germe de maïs, l'huile de germe de riz, l'huile de colza, l'huile d'arachide, l'huile de palme, des terpènes de citron, des terpènes d'orange et des mélanges de ces composants.

7. Émulsion selon l'une des revendications 1 à 6, selon laquelle une substance active hydrophobe est dissoute dans la phase lipidique, dans une concentration comprise entre 0,1 et 70 % en poids, mesurée par rapport à la phase lipidique, laquelle substance active hydrophobe est dispersée.

8. Émulsion selon l'une des revendications 1 à 7, selon laquelle la substance active hydrophobe est un colorant ou une substance active, qui est en particulier sélectionné(e) à partir du groupe constitué par des vitamines, des substances végétales secondaires, l'astaxanthine, la canthaxanthine, la zéaxanthine, le bêta-carotène, des mélanges sélectionnés à partir d' α et ß-carotène, de l'apo carotinal, du lycopène, de la lutéine, de la chlorophylle de cuivre, de la chlorophylline de cuivre, de la curcumine ou de l'extrait de paprika, des acides gras polyinsaturés, de l'alpha tocophérol, des tocotriénols, du xanthohumol, du resvératrol, de la vitamine D, de la vitamine K et des mélanges de ces composants.

9. Émulsion selon l'une des revendications 1 à 8, selon laquelle les divers ingrédients sont sélectionnés à partir des acidifiants, des antioxydants et des agents de conservation, en particulier l'acide citrique, le tocophérol, le palmitate d'ascorbyle, l'acide ascorbique, le sorbate de potassium et le benzoate de sodium.

10. Procédé destiné à la production d'une émulsion comprenant le mélange et la dispersion d'eau, ainsi que :
• 1 à 50 % en poids, mesurés par rapport à l'émulsion, d'une phase lipidique ;
• 1 à 10 % en poids, mesurés par rapport à l'émulsion, de saponine ;
• 5 à 40 % en poids, mesurés par rapport à l'émulsion, de polyols ;
• 5 à 60 % en poids, mesurés par rapport à l'émulsion, d'hydrates de carbone, de préférence 5 à 40 % en poids ou 10 à 60 % en poids ;
• 1 à 30 % en poids, mesurés par rapport à l'émulsion, d'hydrocolloïdes, selon lequel les hydrocolloïdes sont sélectionnés à partir du groupe constitué par la gomme arabique, la gomme arabique modifiée, la gomme ghatti, la gomme ghatti modifiée et des mélanges de ces composants ;
• 0 à 10 % en poids, mesurés par rapport à l'émulsion, de divers ingrédients.

11. Boisson obtenue à partir du mélange compris entre 0,01 et 5 g/l de l'émulsion selon l'une des revendications 1 à 9 avec de l'eau, ainsi que divers autres ingrédients, le cas échéant.

12. Boisson selon la revendication 11, **caractérisée en ce que** la boisson est claire, c'est-à-dire qu'elle présente une turbidité inférieure à 20 FNU.

13. Procédé destiné à la production d'une boisson selon l'une des revendications 11 à 12 comprenant le mélange de 0,01 à 5 g/l d'une émulsion selon l'une des revendications 1 à 9 ainsi que divers autres ingrédients.

14. Base pour boisson, aliment pour animaux, produit pharmaceutique ou produit cosmétique, contenant l'émulsion selon l'une des revendications 1 à 9.
